# EUROPEAN PATENT SPECIFICATION

(11) **EP 3 115 053 B1**
(45) Date of publication and mention of the grant of the patent: **25.11.2020**
(21) Application number: 15758168.7
(22) Date of filing: 03.03.2015
(51) Int. Cl.: A61K 35/54, A61P 5/00

(54) **METHOD FOR PRODUCING FOLLICULAR PROTEIN PREPARATION, AND PREPARATION PRODUCED USING SAID METHOD**
VERFAHREN ZUR HERSTELLUNG EINER FOLLIKELPROTEINZUBEREITUNG UND ANHAND DIESES VERFAHRENS HERGESTELLTE ZUBEREITUNG
PROCÉDÉ DE PRODUCTION D'UNE PRÉPARATION DE PROTÉINE FOLLICULAIRE ET PRÉPARATION OBTENUE À L'AIDE DUDIT PROCÉDÉ

(30) Priority: 03.03.2014 RU 2014107790
(43) Date of publication of application: 11.01.2017
(73) Proprietor: Zakrytoe Akcionernoe Obschhestvo "SKAJ Ltd.", g. Barnaul 656015 (RU)
(72) Inventor: IL'ICHJOV, Aleksandr Vladimirovich, Moscow 105484 (RU); MAL'DOV, Dmitrij Grigor'evich, Vidnovskij r-on Moskovskaja obl. 142784 (RU)
(74) Representative: Cabinet Chaillot
(86) International application number: PCT/RU2015/000128
(87) International publication number: WO 2015/133934

(56) References cited:
- EA-A1- 200 501 217
- RU-C2- 2 201 241
- Anonymous: "IccledoBatelbckii ihctityt pazBitiya i bezopachocti - Research Institute for Development and Security", , 13 December 2013 (2013-12-13), pages 1-9, XP055398184, Retrieved from the Internet: URL:http://web.archive.org/web/20140212163 003/http://www.rids.by/programmy/medicina/ [retrieved on 2017-08-11]
- Anonymous: "Research Institute for Development and Security", , 13 December 2013 (2013-12-13), pages 1-8, XP055398188, Retrieved from the Internet: URL:https://translate.google.de/translate? hl=de&sl=ru&tl=en&u=http://web.archive.org /web/20131213121208/http://www.rids.by/pro grammy/medicina/ [retrieved on 2017-08-11]
- K SUGINO ET AL: "Purification and characterization of high molecular weight forms of inhibin from bovine follicular fluid.", ENDOCRINOLOGY, vol. 130, no. 2, 1 February 1992 (1992-02-01), pages 789-796, XP055398330, US ISSN: 0013-7227, DOI: 10.1210/endo.130.2.1733725
- DE VOS F L ET AL: "Effect of mass loadability, protein concentration and n-alkyl chain length on the reversed-phase high-performance liquid chromatographic behaviour of bovine serum albumin and bovine follicular fluid inhibin", JOURNAL OF CHROMATOGRAPHY A, ELSEVIER, AMSTERDAM, NL, vol. 392, 1 January 1987 (1987-01-01), pages 17-32, XP026487368, ISSN: 0021-9673 [retrieved on 1987-01-01]
- ANDERSEN M M ET AL: "PROTEIN COMPOSITION IN THE FLUID OF INDIVIDUAL BOVINE FOLLICLES", JOURNAL OF REPRODUCTION AND FERTI, JOURNALS OF REPRODUCTION AND FERTILITY LTD, GB, vol. 48, no. 1, 1 September 1976 (1976-09-01), pages 109-118, XP008028634, ISSN: 0022-4251, DOI: 10.1530/JRF.0.0480109
- Proefschriff Ter Verkruging ET AL: "Inhibin - purification and characterization", , 1 January 1986 (1986-01-01), page FP1-6, 1-148, XP055398386, Retrieved from the Internet: URL:https://repub.eur.nl/pub/39029/861112_ DIJK, Simon van.pdf [retrieved on 2017-08-11]
- V. V. SOVA ET AL.: 'Vydelenie i ochistka belkov, Metodicheskoe posobie po kursu «KHmia i biokhimiia belkov i fementov»' VLADIVOSTOK 2006, pages 10 - 11, XP008185029
- 'ENDOFERIN' INFORMATSIONNAYA ZAPISKA, OO «ISSLEDOVATELSKY INSTITUT RAZVITIA I BEZOPASNOSTI» SOVMESTNO S ZAO ''SKAI LTD 2013, pages 7 - 11, XP008184883
- D.V.AKSENENKO: 'KOMPLEKSNOE LECHENIE BESPLODIA PRI NARUZHNOM GENITALNOM ENDOMETRIOZE S ISPOLZOVANIEM TSITOKINOVYKH PREPARATOV' AVTOREFERAT DISSERTATSII 2010, XP008184651

## Description

### Pertinent Art

The invention refers to the field of biological chemistry and may find production application to obtain, from the organic material, protein preparations that can be used in medicine to repress the immunity during transplanting interventions, in the treatment of endometriosis and some oncological diseases, and in veterinary.

Recent investigations have shown an elevated frequency of endometriosis in people carrying mutations in genes coding inhibin βA [Lin J., L. Zong, S.H. Kennedy, K.T. Zondervan Coding regions of INHBA, SFRP4 and HOXA10 are not linked to chromosome 7p13-15 // Molecular Human Reproduction. 2011, V.17, No.10, P.605-611], and some data on the suppression of development of this disease in the mouse model of endometriosis can be found in the literature [Bristol-Gould SK, Hutten CG, Sturgis C, Kilen SM, Mayo KE, Woodruff TK. - The development of a mouse model of ovarian endosalpingiosis. // Endocrinology. 2005; 146(12):5228-36].

Besides, there are data about increased quantities of activin A (a protein of the TGF-β family), which is a physiological antagonist of inhibin in the body of women suffering from endometriosis, in atypical endometrium [Rombauts L., J. Donoghue, L. Cann, R.L. Jones and D.L. Healy - Endometrial activin-A secretion in endometriosis Activin-A secretion is increased in the eutopic endometrium from women with endometriosis.// Australian and New Zealand Journal of Obstetrics and Gynaecology 2006; 46: 148-153].

Both types of proteins act by rendering influence on the activity of sex steroid hormones: activins intensify the activity of estrogens while inhibins suppress it [Peng C, Ohno T, Khorasheh S, Leung PC., - Activin and follistatin as local regulators in the human ovary.// Biol Signals. 1996 Mar-Apr; 5(2):81-9; Knight PG, Glister C., - Local roles of TGF-beta superfamily members in the control of ovarian follicle development.// Anim Reprod Sci. 2003 Oct 15; 78(3-4):165-83].

The document published on 13-12-2013 and found on http://web.archive.org/web/20140212163003/http://www.rids.by/ programmy/medicina/ discloses Endopherin(R) by SKY LTD, a protein drug of natural origin for the treatment of endometriosis, with an active principle based on TGF-β-high-molecular inhibin which is in latent form, activated in a special, undisclosed manner.

Therefore, it is very important to produce a preparation that is free of any components rendering opposite effects when the preparation is used for therapeutic purposes.

### Prior Art

A conventional technique to obtain the follicular regulatory protein is known, which comprises the usage of the follicular fluid as the feedstock, which is isolated from pig ovaries and then sedimented with ammonium sulfate. The fractions so obtained are passed through Orange A gel balanced with Tris-HCl buffer solution with pH 7.5. The bound fractions are eluted with 0.5 M KCl in the same buffer solution. The fractions containing aromatase inhibitors are collected (according to Ohno), dialyzed against distilled water, and freeze-dried. Thereafter, the obtained product is purified using an anion exchange column with mono Q, then the fractions having TGF activity are eluted and purified through gel filtration using Spherogel TSK G 300000 W6 in water via high-performance liquid chromatography. The resultant follicular regulatory protein is concentrated by ultra-filtration using Amicon Dia-flow system with P10 (US 5102867 A, 07.04.1992).

As a result of analysis of the conventional technique, one should note that it is characterized by complexity of implementation, high labor intensity, which makes difficult its implementation in the industry. Besides, the conventional technique does not allow isolating from the follicular regulatory protein composition the specific components that can effectively render a positive treatment effect, for example, targeting endometriosis. The preparation produced by that technique is characterized by weak therapeutic effect.

A technique for obtaining proteins from the bovine follicular fluid is known, according to which the follicular fluid is subjected to sedimentation and the sediment is freeze-dried, moreover, prior to sedimentation, the follicular fluid should be cooled and centrifuged for 18 - 25 minutes.

Sedimentation of impurities is performed with acetone cooled down to -18°C, which is added to the follicular fluid in a ratio of components v/v: follicular fluid - 2, acetone - 3. The resultant suspension is settled and centrifuged at 1000 rpm for 20-35 minutes; subsequently, the sediment is collected and freeze-dried (RU 2185180 C2, 20.07.2002).

As a result of analysis of the known technique, it should be noted that this technique produces a high content of impurities in the ready preparation including activins promoting endometriosis and, hence, cannot be used efficiently for this disease, that is, it has a limited area of application.

A technique for purification of proteins of the transforming growth factors family obtained from bovine follicular fluid is known, according to which protein is dissolved in a buffer solution with neutral pH, and the solution is filtered through a nitrocellulose filter. The resultant supernatant undergoes ion exchange chromatography. Thereafter, the component produced by ion exchange chromatography is passed through an 8-mm column (Cybracon by ICN) with the same buffer, and the active fraction is collected and purified using gel filtration. The resultant product is filtered through UPM 200 filter, and the filtrate is collected. After that, a two-step purification of the filtrate is carried out. At the first step, purification of the filtrate is performed using reverse-phase high-pressure chromatography. At the second step of filtrate purification, preparative isoelectric focusing is engaged (RU 2201241 C2, 27.03.2003) - the closest analogue.

As a result of analysis of the known technique, it should be noted that the technique is characterized by complexity of implementation and high labor intensity hindering its industrial implementation. Besides, the technique does not allow obtaining specifically, among the transforming growth factors, the curative components that effectively target endometriosis.

### Disclosure of the Invention

The technical result of the invention is the development of a simple and available technique for the production of a protein preparation with the specific composition, which secures high biological activity of the preparation and its resistance to environmental factors (temperature, humidity and so on), thus providing a longer shelf life compared to the known techniques.

The said technical result is provided by that in the new technique for obtaining the follicular protein preparation, according to which bovine follicular fluid is collected, which is first centrifuged at 8000-10000 g to produce the supernatant, which is cooled down to + 4°C to + 8°C, that is then mixed with acetone cooled down to -18°C in 1/1 to 2/3 ratio by volume and again centrifuged at 8000 to 10000 g for 15-25 minutes, and the resultant precipitate is freeze-dried, then the lyophilizate is dissolved in 7.2 pH buffer, the resultant suspension is centrifuged again at 8000-10000 g for 15 - 30 min to obtain the supernatant, which is separated using DEAE-Sepharose ion exchange chromatography, the effluent yielding at NaCl concentration of 0.11 M to 0.19 M in the eluting solution having pH 7.0-7.2 is collected and dialyzed against water through a membrane with 14 kDa exclusion volume for at least 8 hours at at temperature of 4 to 8°C, the resultant dialyzate is frozen at a temperature of -18 to -35°C and then freeze-dried to produce the preparation containing serum albumin and inhibin βA, a protein of TGF-β family.

The follicular protein preparation obtained from the bovine follicular fluid contains serum albumin and inhibin βA, a protein of the TGF-β family (TGF-β, transforming growth factor), at the following ratio of their components, % w/w:
- inhibin βA, a protein of the TGF-β family: 0.005;
- impurities: about 2;
- serum albumin: the rest.

Utilization, as feedstock for obtaining the follicular protein preparation, of the follicular fluid isolated from bovine ovarian follicles is essential because: firstly, it ensures a readily available source, and, secondly, according to investigations, the use of follicular fluids from other animal species as feedstock does not allow producing a protein product with the necessary content of active components. According to experimental data, utilization for the production of a follicular protein preparation, for example, of pig follicular fluid (US patent No. 5102867) does not allow obtaining a product with the specified protein composition.

Fluid cooling prior to the first centrifuging allows keeping proteins intact in their native state and increasing their yield.

Centrifuging (both first and second) provides purification of soluble proteins from cellular residue.

Carrying out sedimentation in cooled acetone allows purifying the preparation from non-protein components.

The DEAE-Sepharose ion exchange chromatography results in obtaining a follicular protein preparation of specified composition consisting of inhibin βA, TGF-β family protein and bovine serum albumin.

Term "inhibin βA" is commonly used. Its identical designation - inhibin A - can also be found in the literature.

Lyophilization of the follicular protein preparation allows longer storage of the preparation.

It should be noted and it has been proven experimentally, that the claimed technical result can be achieved only due to unconditional completion of all actions documented in the application materials, strictly adhering to their claimed sequence and modes and observing quantitative ratios of the preparation components. So, the inventive features stated in the claims and the sequence of their completion are essential.

### Proposal Implementation Options

The technique for the production of follicular protein preparation is realized in several steps.

Initially, a bovine follicular fluid is collected from ovaries. The collected fluid is centrifuged at 8000-10000 g to produce the supernatant. The supernatant produced as a result of centrifuging is poured down into a clean container and cooled down to 4 - 8°C in order to augment the quantity of sedimentary protein. Thereafter, acetone (ASC grade) cooled down to -18°C, is added to the supernatant in 1/1 to 2/3 ratio by volume, mixed and centrifuged for 15-25 min. at 8000-10000 g to produce sediment. The supernatant is discarded. The sediment is transferred into a container, cooled down to -18°C and dried in the freeze-drier (VirTis benchtop SLC) until it becomes fully dried. As a result, a lyophilized product in the form of granulated powder of white to dark yellow color, poorly soluble in water, is obtained.

At the next step, the lyophilized product is dissolved in 7.2 pH buffer under continuous stirring for 15-30 min. The resultant suspension is centrifuged at 8000-10000 rpm for 15 - 30 min (Eppendorf 5804 centrifuge). to produce the supernatant. The produced supernatant is poured out and applied onto a DEAE-Sepharose column. The column filling takes 6 - 8 hours. The column is balanced with the buffer for 12 - 24 hours until pH equals to 7-7.2.

The subsequent isolation of the protein preparation can be carried out in two ways yielding the same result:
1. The supernatant obtained from 3 g of the lyophilized follicular fluid is applied onto the column and 180 ml of the buffer is flowed. Using a BioRad BioLogic LP chromatography system with UV detector for λ=280 nm wavelength, 112 ml of 0.11 M NaCl solution (2.6 column volumes, approximately) are fed. Then, 120 ml of 0.11-0.19 M NaCl solution with the buffer is fed and simultaneously collection of the effluent into a container is started. Thereafter, 40 ml of 2M NaCl solution is fed to the column to remove residual applied substances from the column. The collected effluent is frozen in the freezer (Sanyo MDF-436) at - 18°C - 35°C, and then freeze-dried.
2. The supernatant obtained from 3 g of the lyophilized follicular fluid is applied onto the column and eluted with NaCl gradient of 0 to 1 M in 7.05-7.20 pH buffer using a chromatography system (such as BioRad). The effluent washed with 0.11 - 0.19 M NaCl salt solutions is collected. The control is effected using a self-recorder. The collected effluent is frozen in the freezer (Sanyo MDF-436) at -18°C - 35°C, and then freeze-dried.

As a result of either process 1 or process 2, the same effluent is obtained; hence, the expediency of using one or the other process depends on the instrumental stock and technological conditions.

As a result of the described manipulations, the protein preparation with buffer - light-weight airy powder of white color is produced.

To remove the residual buffer contaminant from the produced preparation, at the next step the protein preparation with the buffer is subjected to dialysis, to which end:
- it is dissolved in distilled water;
- dialysis through membrane (ROTH) with a rejection volume of 14 kDa against 2 liters of distilled water is performed, the distillate being replaced according the following schema:
   ∘ dialysis for 3 hours (2 liters of distillate);
   ∘ replacement of distillate;
   ∘ dialysis for 3 hours (2 liters of distillate);
   ∘ replacement of distillate;
   ∘ dialysis for 2 hours (2 liters of distillate).

The process is carried out at 4 - 8°C.

The dialysate is produced. The membrane is cut and the solution is poured out into a glass beaker and freeze-dried.

The quantitative content of the components stated in the claims is checked using the common ELISA technique, and activity of the protein preparation is checked using the standard method of determining biological activity on VERO cell culture.

As a result of this technique realization, the follicular protein preparation is obtained, which contains serum albumin and inhibin βA, a protein of the TGF-β family, with the components ratio as follows, % by weight:
- inhibin βA, a protein of the TGF-β family featuring inhibin βA immune specificity: 0.005;
- impurities: about 2;
- serum albumin: the rest.

The essence of the claimed procedure will be easier to understand from the example given. Initially, bovine follicular fluid is collected, to which end, bovine ovaries in the follicles area are rubbed with 96% alcohol, then punctured with a disposable sterile syringe, and 10 ml of the follicular fluid is collected into an alcohol-rinsed centrifuge test-tube having a volume of not less than 20 ml. The follicular fluid is centrifuged for 25 minutes at 8000 g (Eppendorf 5804 centrifuge). The resultant supernatant is poured out into a clean container and cooled to 6°C. Then, acetone (ASC grade), cooled to -18°C, is added to the supernatant in 1/1 ratio by volume, mixed and centrifuged for 15 min. at 8000 g (Eppendorf 5804 centrifuge). The supernatant is discarded. The sediment is transferred into a container, cooled to -18°C, and dried in a freeze-drier (VirTis benchtop SLC) until it is fully dried.

Thereafter, 1.3 g of the lyophilized follicular fluid is dissolved in 40 ml of 7.2 pH buffer in a 50-ml glass flask using ultrasound stirrer UZM 001 (Poland) under continuous stirring for 30 minutes. The resultant suspension is centrifuged using Eppendorf 5804 centrifuge at 8000 rpm for 30 minutes. The supernatant (approx. 8 ml) is poured out and applied onto a column (cat. No. 57407-08-6 DFF100, Sigma, Diethylaminoethyl Sepharose. Column: glass, 1.5^{∗}30 cm in size, the effective volume is 42 ml. The column filling time is 6 hours. The time of column balancing with the buffer to pH 7.2 is 12 hours). After that, the supernatant produced from 1.3 g of the lyophilized follicular fluid is applied onto the column, and the column is washed with 180 ml of the buffer. Using the chromatography system (BioRad BioLogic LP with UV detector for λ=280 nm wavelength), 112 ml of 0.11 M NaCl solution (2.6 column volumes, approximately) are fed. Then, 120 ml of 0.19 M NaCl solution in the buffer is fed and simultaneously collection of the effluent into a container is started (120 ml of solution should be obtained). Thereafter, 40 ml of 2 M NaCl solution is fed to the column to remove residual applied substances from the column. The collected effluent is frozen in a freezer (Sanyo MDF-436) at - 35°C, and then freeze-dried (VirTis benchtop SLC).

After that, dialysis of the resultant lyophilisate is performed, to which end:
- 0.26 g of the protein is dissolved in 10 ml of distilled water.
- dialysis through a membrane (ROTH) is carried out with the rejection volume of 14 kDa against 2 liters of distilled water, the distillate being changed according to the following scheme:
   ∘ dialysis for 3 hours (2 liters of distillate);
   ∘ replacement of distillate;
   ∘ dialysis for 3 hours (2 liters of distillate);
   ∘ replacement of distillate;
   ∘ dialysis for 2 hours (2 liters of distillate).

The process is carried out at 6°C.
12 ml of dialysate is obtained. The membrane is cut and the solution is poured out into a 50 ml glass beaker. Protein concentration is established according to Bradford, and the freeze-drying procedure is repeated.

The contents of the components stated in the claims are checked using ELISA technique, and the protein preparation activity is checked by determining biological activity using VERO cell culture.

As a result, 0.26 g of the follicular protein preparation is obtained, which contains:
- bovine serum albumin 0.254813 g
- inhibin βA, a protein of the TGF-β family 0.000013 g
- impurities 0.005174 g, which do not render significant influence on the said technical result.

It should be noted that the said technical result can be only achieved within the limits of contents of the components of the follicular protein preparation as stated in the claims.

In case of lower contents of inhibin βA, a TGF-β family protein, the preparation loses its activity; In case of higher contents, the risk of occurrence of pathological side effects rises. The presence of serum albumin provides stability of inhibins in the protein preparation.

This preparation was verified on the rat model of endometriosis. Intraperitoneal administration of the preparation dosed 40 µg per animal one dose daily for 10 days to rats with surgically induced endometriosis led to 7.9-fold reduction of the implant volume in these rats.

### Industrial Applicability

The preparation produced by this technique and having the afore-stated composition was applied in the treatment of endometriosis in women.

Endometriosis is a common gynecological disease when the endometrium (the inner layer of the uterus wall) cells grow beyond that layer. The endometrioid tissue has receptors to hormones; hence, it is prone to the same changes that occur in normal endometrium and reveal themselves in monthly bleeding. These small bleedings result in inflammation in the surrounding tissues and cause the main manifestations of the disease: pain, increased volume of the organ, infertility. The main methods of the current endometriosis treatment are hormonal therapy and surgery.

### Examples.

The treatment was performed with the preparation (dosed 0.3 mg) with the following composition:
- bovine serum albumin 0.25 mg
- inhibin βA, a protein of the TGF-β family featuring inhibin βA 0.000013 mg

The therapeutic dosage regimen:
- the first month - 10 injections, 0.3 mg each, every day beginning from day five of the monthly cycle.
- the second month - 10 injections, 0.3 mg each, every day beginning from day five of the monthly cycle.
- the third month - 10 injections, 0.3 mg each, every day beginning from day five of the monthly cycle.

Patient U., 27 years old. Clinical diagnosis: uterus endometriosis, diffuse form, diagnosed in 2008. Complaints of heavy painful menstrual fluxes.

Clinical and biochemical indices prior to the therapy: urinalysis, total blood count, biochemistry, coagulogram, tumor markers are within normal limits. Blood hormone levels: estradiol - 0.618 nmol/l, progesterone - 7.6 nmol/l. Ultrasound examination of pelvic organs: uterus endometriosis. Morphological conclusion: active adenomyosis.

Positive dynamics registered at the background of therapy: no pain, moderate menstrual flux. The clinical and biochemical indices are within normal limits. In the hormonal blood analysis, increase of estradiol up to 0.72 nmol/l, decrease of progesterone down to 3.97 nmol/l is noted. Ultrasound examination of pelvic organs: positive dynamics. Morphological conclusion: endometriosis is not found (positive dynamics).

Patient S., 39 years old. Clinical diagnosis: uterus endometriosis, focal, diagnosed in 2002. Complaints of heavy painful menstrual fluxes.

Clinical and biochemical indices prior to the therapy: urinalysis, biochemistry, coagulogram, tumor markers are within normal limits. Total blood count: Hb - 110g/l. Tumor markers CA 125 - 47.6 (the norm is less than 35 U/ml). Blood hormones: estradiol - 0.56 nmol/l, progesterone - 1.8 nmol/l. Ultrasound examination of pelvic organs: uterus endometriosis. Morphological conclusion: moderately active adenomyosis.

Positive dynamics at the background of the therapy: pain and hyperpolymenorrhea syndrome decreased. The clinical and morphological indices are within normal limits. Clinical blood analysis: Hb - 102 g/l. In the hormonal blood analysis, increase of estradiol level up to 0.8 nmol/l, decrease of progesterone level to 0.2 nmol/l is noted. Ultrasound examination of pelvic organs: positive dynamics. Morphological examination: weakly active endometriosis (positive dynamics).

Patient K., 33 years of age. Clinical diagnosis: uterus endometriosis, diffuse form, diagnosed in 2000. Complaints of heavy menstrual fluxes. Irregular menstrual cycle.

Clinical and biochemical indices prior to the therapy: urinalysis, total blood count, biochemistry, coagulogram, tumor markers are within normal limits. Blood hormones: estradiol 0.538 - nmol/l, progesterone - 3.7 nmol/l. Ultrasound examination of pelvic organs: uterus endometriosis. Morphological conclusion: weakly active adenomyosis.

Positive dynamics at the background of therapy is noted: the hyperpolymenorrhea syndrome decreased. The clinical and morphological indices are within normal limits. In the hormonal blood analysis, decrease of estradiol down to 0.218 nmol/l, increase of progesterone up to 36.7 nmol/l is noted. Ultrasound examination of pelvic organs: positive dynamics. Morphological examination: endometriosis is not found (positive dynamics).

No side effects were observed in the course of treatment.

Preparation administration to patients suffering from endometriosis has led to: disappearance of hyperpolymenorrhea - 8% of cases, and its decrease - 92% of cases; disappearance of algodismenorrhea - 36% of cases, and its decrease - 64% of cases.

In assessment of the disease severity - symptoms disappeared in 32% of cases and severity decreased in 68% of cases.

Based on the conclusion of the comprehensive (histological and immunomorphological) examination, the positive effect of the therapy was established in 80% of cases, and in the remaining cases the trend towards reduced activity of adenomyosis foci was found.

The preparation can also be used for the treatment of some oncological diseases and in veterinary.

## Claims

1. A method for the production of follicular protein preparation, according to which bovine follicular fluid is collected, which is first centrifuged at 8000-10000 g to produce the supernatant, which is cooled down to + 4°C to + 8°C, that is then mixed with acetone cooled down to -18°C in 1/1 to 2/3 ratio by volume and again centrifuged at 8000 to 10000 g for 15-25 minutes, and the resultant precipitate is freeze-dried, then the lyophilizate is dissolved in 7.2 pH buffer, the resultant suspension is centrifuged again at 8000-10000 g for 15 - 30 min to obtain the supernatant, which is separated using DEAE-Sepharose ion exchange chromatography, the effluent yielding at NaCl concentration of 0.11 M to 0.19 M in the eluting solution having pH 7.0-7.2 is collected and dialyzed against water through a membrane with 14 kDa exclusion volume for at least 8 hours at at temperature of 4 to 8°C, the resultant dialyzate is frozen at a temperature of -18 to -35°C and then freeze-dried to produce the preparation containing serum albumin and inhibin βA, a protein of TGF-β family.

2. A follicular protein preparation obtainable from the method according to claim 1 for use in the treatment of endometriosis, wherein the preparation contains serum albumin and inhibin βA, a protein of the TGF-β family, at the following ratio of their components, % by weight:
- inhibin βA, a protein of the TGF-β family, 0.005;
- impurities: about 2;
- serum albumin: the remainder.

## Patentansprüche

1. Verfahren zum Herstellen einer Follikelproteinzubereitung, gemäß welchem Rinderfollikelflüssigkeit gesammelt wird, die zuerst bei 8000 bis 10.000 g zentrifugiert wird, um den Überstand herzustellen, der auf +4 °C bis +8 °C abgekühlt wird, danach mit Aceton vermischt wird, auf -18 °C in einem Volumenverhältnis von 1/1 bis 2/3 abgekühlt und danach erneut für 15 bis 25 Minuten bei 8000 bis 10.000 g zentrifugiert wird, und das entstehende Präzipitat gefriergetrocknet wird, danach das Lyophilisat in einem Puffer mit einem pH-Wert von 7,2 gelöst wird, die entstehende Suspension erneut bei 8000 bis 10.000 g für 15 bis 30 min zentrifugiert wird, um den Überstand zu erhalten, der unter Verwendung von DEAE-Sepharose-Ionenaustauschchromatographie getrennt wird, wobei das abfließende Produkt, das in einer NaCl-Konzentration von 0,11 M bis 0,19 M in der eluierenden Lösung mit einem pH-Wert von 7,0 bis 7,2 produziert wird, gesammelt und für zumindest 8 Stunden bei einer Temperatur von 4 bis 8 °C gegen Wasser durch eine Membran mit einem Ausschlussvolumen von 14 kDa dialysiert wird, wobei das entstehende Dialysat bei einer Temperatur von -18 bis -35 °C gefroren und danach gefriergetrocknet wird, um die Zubereitung herzustellen, die Serumalbumin und Inhibin βA, ein Protein der TGF-β-Familie, enthält.

2. Follikelproteinzubereitung, die mit dem Verfahren nach Anspruch 1 erhältlich ist, zur Verwendung bei der Behandlung von Endometriose, wobei die Zubereitung Serumalbumin und Inhibin βA, ein Protein der TGF-β-Familie, in dem folgenden Verhältnis ihrer Komponenten in Gewichtsprozent enthält:
- Inhibin βA, ein Protein der TGF-β-Familie, 0,005;
- Verunreinigungen: ungefähr 2;
- Serumalbumin: der Rest.

## Revendications

1. Procédé de production d'une préparation de protéine folliculaire, selon lequel du fluide folliculaire bovin est collecté, lequel est d'abord centrifugé à 8000 - 10000 g pour produire le surnageant, qui est refroidi jusqu'à + 4°C à + 8°C, qui est ensuite mélangé avec de l'acétone refroidie jusqu'à -18°C dans un rapport de 1/1 à 2/3 en volume et à nouveau centrifugé à 8000 à 10000 g pendant 15 - 25 minutes, et le précipité résultant est lyophilisé, puis le lyophilisat est dissous dans un tampon pH à 7,2, la suspension résultante est centrifugée à nouveau à 8000 - 10000 g pendant 15 - 30 minutes pour obtenir le surnageant, qui est séparé par chromatographie d'échange d'ions DEAE-Sepharose, l'effluent étant produit à une concentration de NaCl de 0,11 M à 0,19 M dans la solution d'élution ayant un pH de 7,0 - 7,2 est collecté et dialysé contre de l'eau à travers une membrane ayant un volume d'exclusion de 14 kDa pendant au moins 8 heures à une température de 4 à 8°C, le dialysat résultant est congelé à une température de -18 à -35°C puis lyophilisé pour produire la préparation contenant de la sérum albumine et de l'inhibine βA, une protéine de la famille des TGF-β.

2. Préparation de protéine folliculaire pouvant être obtenue à partir du procédé selon la revendication 1 pour une utilisation dans le traitement de l'endométriose, la préparation contenant de la sérum albumine et de l'inhibine βA, une protéine de la famille des TGF-β, dans le rapport suivant de leurs composants, en % en poids :
- inhibine βA, une protéine de la famille des TGF-β : 0,005 ;
- impuretés : environ 2 ;
- sérum albumine : le reste.
